# EUROPÄISCHE PATENTSCHRIFT

(11) **EP 1 400 223 B1**
(45) Veröffentlichungstag und Bekanntmachung des Hinweises auf die Patenterteilung: **28.02.2007**
(21) Anmeldenummer: 03018577.1
(22) Anmeldetag: 18.08.2003
(51) Int. Cl.: A61F 13/15

(54) **Verpacktes Hygieneprodukt zur Aufnahme von Körperflüssigkeiten und/oder -ausscheidungen und Verfahren zur Herstellung eines derartigen Produkts**
Packed hygiene product for acquiring body fluids and method of producing such a product
Produit d'hygiène emballé pour collecter les sécrétions corporelles ainsi que son procédé de fabrication

(30) Priorität: 17.09.2002 DE 10243156
(43) Veröffentlichungstag der Anmeldung: 24.03.2004
(73) Patentinhaber: Winkler + Dünnebier Aktiengesellschaft, 56564 Neuwied (DE)
(72) Erfinder: Becker, Albert, 56581 Ehlscheid (DE)

(56) Entgegenhaltungen:
- EP-A- 0 798 229
- EP-A- 1 043 004
- EP-A- 1 157 680
- WO-A-96/20668
- DE-A- 19 903 285
- US-A- 5 484 636
- US-B1- 6 234 229
- US-B1- 6 293 932

## Beschreibung

### I. Anwendungsgeblet

Die vorliegende Erfindung betrifft ein verpacktes Hygieneprodukt zur Aufnahme von Körperflüssigkeiten und/oder -ausscheidungen sowie ein Verfahren zur Herstellung eines derartigen Produktes. Unter Hygieneprodukt im Sinne der vorliegenden Erfindung sind insbesondere Slipeinlagen, Damenbinden, Windeln, Papiertaschentücher und ähnliche Produkte zu verstehen.

### ll. Technischer Hintergrund

Es ist bekannt, zusammengefaltete Damenbinden lediglich mit Hilfe der Seitenprägungen des die Damenbinde umgebenden Verpackungsmaterials in verpacktem Zustand zu halten. Dabei tritt häufig ein Fischmauleffekt auf, bei dem sich das Verpackungsmaterial entlang seiner außen liegenden Stirnseitenkante, welche zwischen den beiden Seitenprägungen verläuft, von demjenigen, unter ihr liegenden Bereich des Verpackungsmaterials, an welchem sie zunächst lose anliegt, aufgrund von Rückstellkräften des Verpackungsmaterials in unerwünschter Weise abhebt. Dadurch entsteht eine tunnelartige, seitlich von den Prägungen begrenzte Öffnung, die ihrem Aussehen nach einem Fischmaul ähnelt.

Eine verpackte Damenbinde, bei welcher der Fischmauleffekt im wesentlichen vermieden wird, ist beispielsweise aus Fig. 11 der US-A 6,234,229 bekannt. Diese zusammengelegte bzw. -gefaltete Damenbinde ist von einem Verpackungsmaterial umgeben, das von einem Klebebandstreifen (Tape) in geschlossenem Zustand gehalten wird. Alternativ zu dem Klebebandstreifen ist es auch bekannt, die einander überlappenden Endbereiche des Verpackungsmaterials mit Hilfe eines separaten Leimauftrages miteinander zu verkleben und dadurch den Fischmauleffekt zu vermeiden.

Die bekannten Endverschlüsse in Form des Klebebandstreifens oder des separaten Leimauftrages vermeiden zwar den Fischmauleffekt, bringen jedoch verschiedene Nachteile mit sich. Sie erfordern zusätzlichen Materialeinsatz, nämlich entweder den Klebebandstreifen (Tape) oder den separaten Leim. Letzteres schlägt sich nicht nur im Preis des verpackten Hygieneprodukts selbst nieder, sondern auch in dem Aufwand, der für Entwicklung und Herstellung der Maschinen zur Herstellung verpackter Hygieneprodukte getrieben werden muß. So benötigen die Maschinen zur Herstellung verpackter Hygieneprodukte mit den herkömmlichen Endverschlüssen entweder eine Station zum Aufbringen des Klebebandstreifens oder eine Station zum Aufbringen des separaten Leimes, jeweils verbunden mit zugehöriger Maschinenlogistik für die Zuführung sowie Vorratshaltung der Klebebandstreifen oder des zusätzlichen Leimes.

### lll. Darstellung der Erfindung

### a) Technische Aufgabe

Es ist daher die Aufgabe der vorliegenden Erfindung, ein verpacktes Hygieneprodukt zur Aufnahme von Körperflüssigkeiten und/oder -ausscheidungen sowie ein Verfahren zur Herstellung eines derartigen Produktes zu schaffen, das einerseits den Fischmauleffekt vermeidet sowie andererseits sowohl das verpackte Hygieneprodukt selbst billiger in seiner Herstellung macht als auch die Maschinen zur Herstellung der verpackten Hygieneprodukte vereinfacht.

### b) Lösung der Aufgabe

Diese Aufgabe wird mit einem verpackten Hygieneprodukt bzw. mit einem Verfahren zur Herstellung eines verpackten Hygieneprodukts mit den Merkmalen des Anspruchs 1 bzw. des Anspruchs 14 gelöst. Weitere Ausgestaltungen der vorliegenden Erfindung ergeben sich aus den Unteransprüchen.

Erfindungsgemäß wird ein verpacktes Hygieneprodukt zur Aufnahme von Körperflüssigkeiten und/oder -ausscheidungen vorzugsweise des menschlichen Körpers vorgeschlagen, welches eine dem menschlichen Körper zugewandte Körperseite sowie eine von dem menschlichen Körper abgewandte Wäscheseite aufweist und zum Verpacken zusammenleg- oder rollbar ist, wobei sich an der Wäscheseite wenigstens teilweise ein Klebemittel befindet, und welches ein Verpackungsmaterial, das eine dem Hygieneprodukt zugewandte Innenseite und eine von dem Hygieneprodukt abgewandte Außenseite aufweist, umfaßt, wobei die Wäscheseite des Hygieneprodukts mit Hilfe des Klebemittels an der Innenseite des Verpakkungsmaterials befestigt ist. Dieses verpackte Hygieneprodukt ist erfindungsgemäß dadurch gekennzeichnet, dass das Verpackungsmaterial wenigstens einen Durchbruch aufweist, durch den hindurch ein Flächenbereich der Innenseite des Verpackungsmaterials mit Hilfe eines Teils des Klebemittels an der Wäscheseite des Hygieneprodukts lösbar befestigbar ist, um das Hygieneprodukt in verpacktem Zustand zu halten.

Bei dem erfindungsgemäßen, verpackten Hygieneprodukt ist die Innenseite des Verpackungsmaterials, bei dem es sich vorzugsweise um eine dünne, flexible Verpackungsfolie handelt, mit Hilfe des Klebemittels lösbar an der Wäscheseite des Hygieneprodukts befestigt. Für den bestimmungsgemäßen Gebrauch des Hygieneprodukts kann das Verpackungsmaterial somit einfach von Hand von der Wäscheseite des Hygieneprodukts abgezogen werden. Die Befestigung des Hygieneprodukts an dem Verpackungsmaterial erfolgt mit Hilfe eines flächigen Leimauftrags vorzugsweise auf der Wäscheseite des Hygieneprodukts. Dieser flächige Leimauftrag kann in einem einzigen, zusammenhängenden Flächenbereich der Wäscheseite angeordnet sein oder mehrere, nicht miteinander zusammenhängende Leimauftragsflächen umfassen.

Das Zusammenlegen des Hygieneprodukts zum verpackten Hygieneprodukt erfolgt erfindungsgemäß vorzugsweise durch Zusammenfalten. Die Erfindung kann insbesondere Anwendung bei den sogenannten "Quick-Wrap"-Produkten finden, bei denen es sich beispielsweise um dreigefaltete Slipeinlagen oder Binden handelt. Dreigefaltet in diesem Sinne bedeutet, dass insgesamt zwei Faltlinien vorhanden sind, d.h. insgesamt zwei Faltungen zum Verpacken des Hygieneprodukts durchgeführt werden, und die beiden Faltlinien das Hygieneprodukt in insgesamt drei Bereiche unterteilen. Alternativ zum Zusammenfalten ist die vorliegende Erfindung jedoch auch anwendbar, wenn das Hygieneprodukt zum Verpacken zusammengerollt oder anderweitig in seiner abgewickelten Grundfläche verkleinert wird.

Bei dem erfindungsgemäßen, verpackten Hygieneprodukt gibt es an der Innenseite des Verpackungsmaterials einen Bereich, der von der Wäscheseite des Hygieneprodukts überdeckt wird. Dieser Bereich wird im folgenden Überdeckungsbereich genannt. Da maximal über die gesamte Wäscheseite des Hygieneprodukts das Klebemittel aufgetragen wird, kann der entsprechende Klebemittelflächenbereich maximal die Größe der Fläche des Überdeckungsbereichs erreichen. Der erfindungsgemäße, wenigstens eine Durchbruch ist in dem Verpackungsmaterial derart angeordnet, dass er in dem Überdeckungsbereich der Innenseite zu liegen kommt. Dabei ist Größe und Geometrie des Durchbruchs so zu wählen, dass derjenige Bereich der Wäscheseite, der durch den Durchbruch hindurch zugänglich ist, zumindest teilweise, vorzugsweise vollständig, mit Klebemittel benetzt ist. Dies gewährleistet, dass nach dem Zusammenlegen bzw. -falten des mit dem Verpackungsmaterial verbundenen Hygieneprodukts ein begrenzter Flächenbereich der Innenseite des Verpackungsmaterials mit mit dem Klebemittel benetzten Bereich der Wäscheseite, der durch den Durchbruch hindurch zugänglich ist, lösbar verklebt werden kann, um das Verpackungsmaterial geschlossen bzw. das Hygieneprodukt insgesamt in verpacktem Zustand zu halten. Dabei befindet sich derjenige Flächenbereich der Innenseite, der mit der Wäscheseite des Hygieneprodukts verklebt wird, außerhalb des Überdeckungsbereichs.

Da bei der herkömmlichen Herstellung verpackter Hygieneprodukte der in Rede stehenden Art ohnehin ein Klebemittel auf die Wäscheseite des Hygieneprodukts aufgetragen wird, um dort das Verpackungsmaterial zu befestigen, nutzt somit die vorliegende Erfindung in vorteilhafter Weise das ohnehin aufzutragende Klebemittel, um die Verpackung des Hygieneprodukts in geschlossenem Zustand zu halten. Zusätzliche Maßnahmen, wie etwa ein Klebebandstreifen (Tape) oder eine separat vorzusehende Zusatzleimung sind erfindungsgemäß daher nicht erforderlich.

Der Durchbruch in dem Verpackungsmaterial kann je nach Anwendungsfall eine Vielzahl von Geometrien aufweisen. Denkbar sind beispielsweise längliche Formen. Weist das nicht zusammengelegte Hygieneprodukt selbst auch eine längliche Form auf, so kann die Längsachse des länglichen Durchbruchs im Wesentlichen quer oder parallel zu der Längsachse des Hygieneprodukts verlaufen. Es ist auch denkbar, dass sich der erfindungsgemäße Durchbruch über die gesamte oder nahezu die gesamte Breite des Hygieneprodukts erstreckt.

Darüber hinaus können auch mehrere Durchbrüche vorgesehen werden, beispielsweise in Form zweier oder mehrerer im Wesentlichen parallel zueinander verlaufender Streifendurchbrüche. Auch Durchbrüche in der Art einer Perforierung, die geradlinig oder entlang einer gekrümmten Kurve verlaufend angeordnet sind, können vorgesehen werden. Im Hinblick auf die Geometrien des Durchbruchs bzw. der Durchbrüche sind insbesondere Rechteckformen, Quadratformen, Kreisformen, Ellipsenformen oder ähnliche Geometrien denkbar. Die Durchbrüche können bei Bedarf sowie bei transparentem Verpackungsmaterial auch in Form einer für den Benutzer durch das Verpackungsmaterial hindurch-lesbaren Gebrauchsinformation angeordnet bzw. einperforiert werden, wie beispielsweise in Form des Schriftzuges "HIER ABZIEHEN".

Bei dem Klebemittel handelt es sich vorzugsweise um Adhäsivleim, der durch Kontakt- oder Sprühauftrag Anwendung findet. Der Sprühauftrag bringt gegenüber dem Kontaktauftrag bei solchen Hygieneprodukten einen Vorteil mit sich, die keine konstante Dicke aufweisen, wie beispielsweise Damenbinden. Bei Sprühauftrag verteilt sich der Leim aufgrund seiner geringeren Viskosität in verhältnismäßig kurzen Zeiträumen gleichmäßig über die Leimauftragsfläche. Dies ist bei Kontaktauftrag nicht immer der Fall. Als Klebemittel könnten auch Teile der Innenseite des Verpackungsmaterials und/oder der Wäscheseite des Hygieneprodukts selbst dienen. Beispielsweise ist eine Ultraschallverschweißung der Innenseite des Verpackungsmaterials mit der Wäscheseite des Hygieneprodukts denkbar. Dabei ist lediglich zu gewährleisten, dass das Verpackungsmaterial von Hand sowie ohne Beschädigung des Hygieneprodukts von Letzterem abziehbar ist.

Bei dem erfindungsgemäßen Verfahren zur Herstellung eines verpackten Hygieneprodukts werden zunächst das Hygieneprodukt selbst sowie das vorzugsweise zugeschnittene Verpackungsmaterial bereitgestellt. Dann werden die Wäscheseite des Hygieneprodukts und die Innenseite des Verpackungsmaterials mit Hilfe des Klebemittels lösbar miteinander verbunden. Vorzugsweise vor dem Befestigen der Wäscheseite des Hygieneprodukts an der Innenseite des Verpackungsmaterials erfolgt das Einbringen, vorzugsweise Ausschneiden, des Durchbruchs in das Verpackungsmaterial. Das Zusammenlegen, -falten oder -rollen des Hygieneprodukts samt daran befestigtem Verpackungsmaterial endet schließlich damit, dass durch den Durchbruch hindurch ein Flächenbereich der Innenseite des Verpackungsmaterials an der Wäscheseite des Hygieneprodukts befestigt wird.

### c) Ausführungsbeispiel

Nachfolgend wird eine Ausführungsform der vorliegenden Erfindung beispielhaft anhand einer dreigefalteten Slipeinlage mit erfindungsgemäßem Endverschluß ohne Zusatzleimung oder Klebebandstreifen (Tape) beschrieben. Es zeigen:
- Fig. 1: eine Ansicht auf die Außenseite des Verpackungsmaterials;
- Fig. 2: eine Aufsicht auf die Wäscheseite der Slipeinlage;
- Fig. 3: eine Schnittansicht gemäß Schnitt A-A in Fig. 4;
- Fig. 4: eine Ansicht auf die mit dem Verpackungsmaterial verbundene Slipeinlage in nicht zusammengefaltetem Zustand;
- Fig. 5A - 5C: verschiedene Momentanzustände, welche die mit dem Verpakkungsmaterial verbundene Slipeinlage während des Zusammenfaltens zeigen;
- Fig. 6: das vergrößerte Detail D gemäß Fig. 7;
- Fig. 7: eine Schnittansicht gemäß Schnitt B-B in Fig. 8; und
- Fig. 8: eine Ansicht auf die fertige, verpackte Slipeinlage gemäß Fig. 5C von oben.

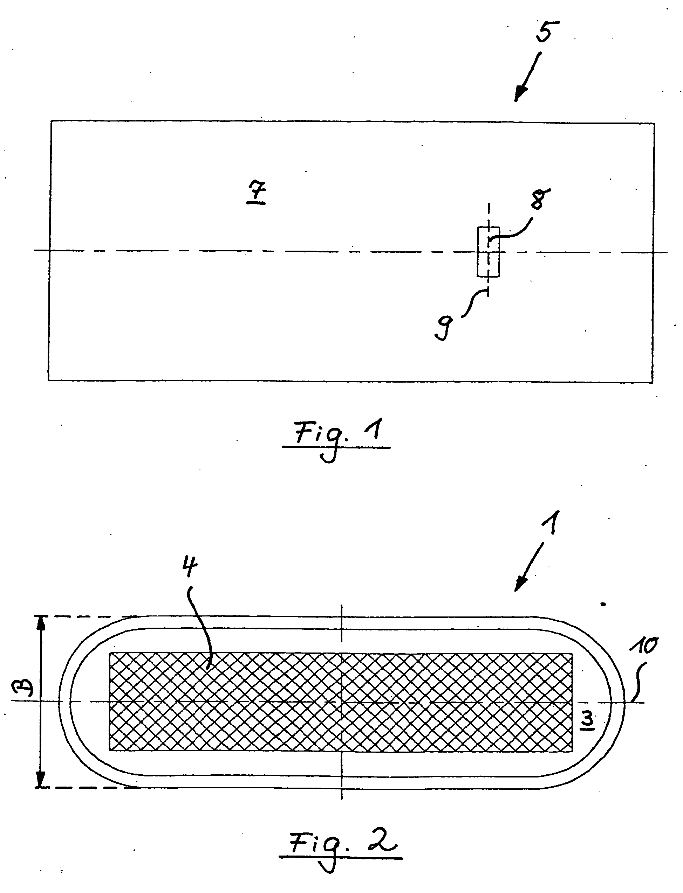

Fig. 1 zeigt eine Ansicht der Außenseite 7 des rechteckförmig zugeschnittenen Verpackungsmaterials 5, bei dem es sich vorzugsweise um eine flexible bzw. elastische Verpackungsfolie aus PE (Polyethylen) oder PP (Polypropylen) geringer Dicke handelt. Übliche Dicken der flexiblen Folie liegen im Bereich von einigen 10 µm. Wie zu erkennen ist, weist die Verpackungsfolie 5 einen länglichen, rechteckförmigen Durchbruch 8 mit einer Längsachse 9 auf. Fig. 2 zeigt eine Ansicht der Wäscheseite 3 einer an sich bekannten Slipeinlage 1 mit abgerundeten Enden. Ein rechteckförmiger Flächenbereich der Wäscheseite 3, der mit einer Kreuzschraffur gekennzeichnet ist, ist durchgängig flächig mit einem Klebemittel 4 benetzt. Bei Bedarf können auch mehrere, nicht zusammenhängende Flächenbereiche der Wäscheseite 3 benetzt werden. Senkrecht zu ihrer Längsachse 10 weist die Slipeinlage 1 die Breite B auf.

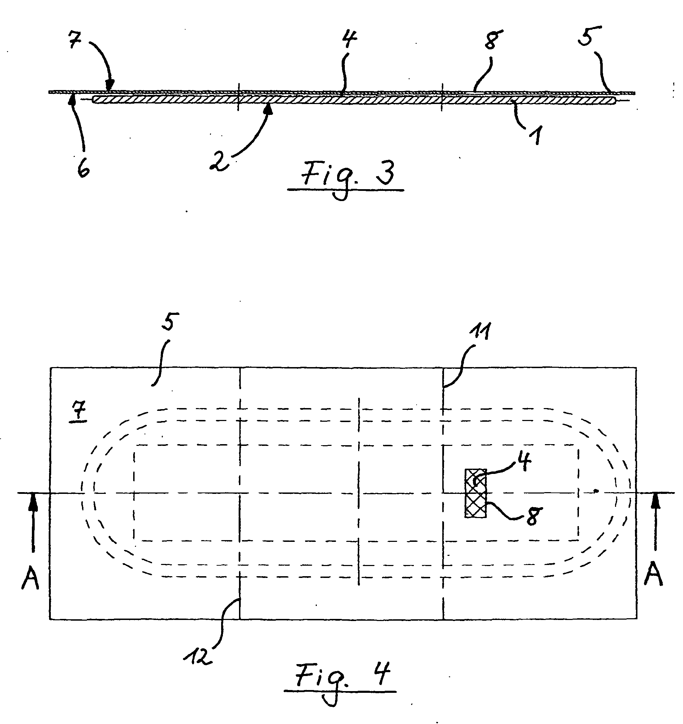

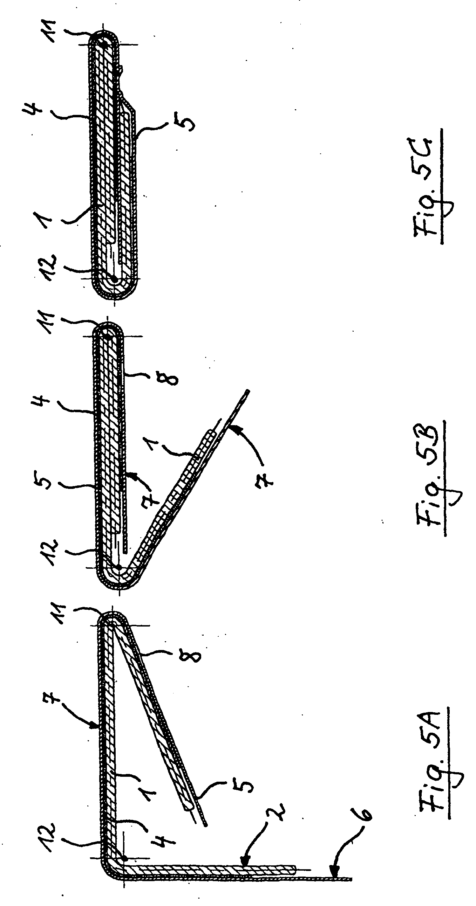

Die Fig. 3 und 4 zeigen die mit der Verpackungsfolie 5 verbundene Slipeinlage 1, wobei die Slipeinlage 1 in der Ansicht gemäß Fig. 4 in gestrichelten Linien dargestellt ist, da sie sich unterhalb der Verpacküngsfolie 5 befindet. Ebenso in gestrichelten Linien ist in Fig. 4 die rechteckförmige Auftragsfläche des Klebemittels 4 gekennzeichnet. Durch den rechteckförmigen Durchbruch 8 hindurch ist ein Teil des Klebemittels 4 in der Blickrichtung der Fig. 4 zugänglich. Die einen Teil der Innenseite 6 der Verpackungsfolie 5 mit einem Teil der Wäscheseite 3 der Slipeinlage 1 verbindende Klebemittelschicht 4 ist insbesondere in der Schnittansicht gemäß Fig. 3 dargestellt. Die mit dem Körper der Benutzerin in Berührung kommende Körperseite 2 der Slipeinlage 1 ist in Fig. 3 ebenso gekennzeichnet. Die Innenseite 6 der Verpackungsfolie 5 ist der Slipeinlage 1 zugewandt während ihr die Außenseite 7 der Verpackungsfolie 5 abgewandt ist. Fig. 4 macht deutlich, dass der Durchbruch 8 innerhalb des Überdeckungsbereiches liegt, in welchem die Slipeinlage 1 die Innenseite 6 der Verpackungsfolie 5 bedeckt.

Die Fig. 5A bis 5C zeigen den Vorgang des Zusammenfaltens der in den Fig. 3 und 4 gezeigten Einheit aus Verpackungsfolie 5, Klebemittelschicht 4 und Slipeinlage 1. Wie in Fig. 5A dargestellt, erfolgt zunächst eine erste Faltung um die erste Faltlinie 11. Dabei werden zwei benachbarte Bereiche der Körperseite 2 der Slipeinlage 1 aneinandergelegt, wie in dem diesbezüglichen Endzustand zu erkennen ist, den Fig. 5B zeigt. Der Durchbruch 8 in der Verpackungsfolie 5 weist einen gewissen Abstand von der ersten Faltlinie 11 auf und wird zusammen mit dem zugehörigen Bereich der Verpackungsfolie 5 um 180° aus der Zeichenebene der Fig. 4 heraus und wieder in diese hineingefaltet. Anschließend erfolgt eine zweite Faltung ebenso um 180° um die zweite Faltlinie 12. Dabei wird der in Fig. 5A linke untere Bereich der Slipeinlage 1 mit seiner Körperseite 2 auf die Außenseite 7 desjenigen Teils der Verpackungsfolie 5 gelegt, der im Rahmen der ersten Faltung um die erste Faltlinie 11 umgefaltet wurde. Der Endzustand der zweiten Faltung ist in Fig. 5C dargestellt.

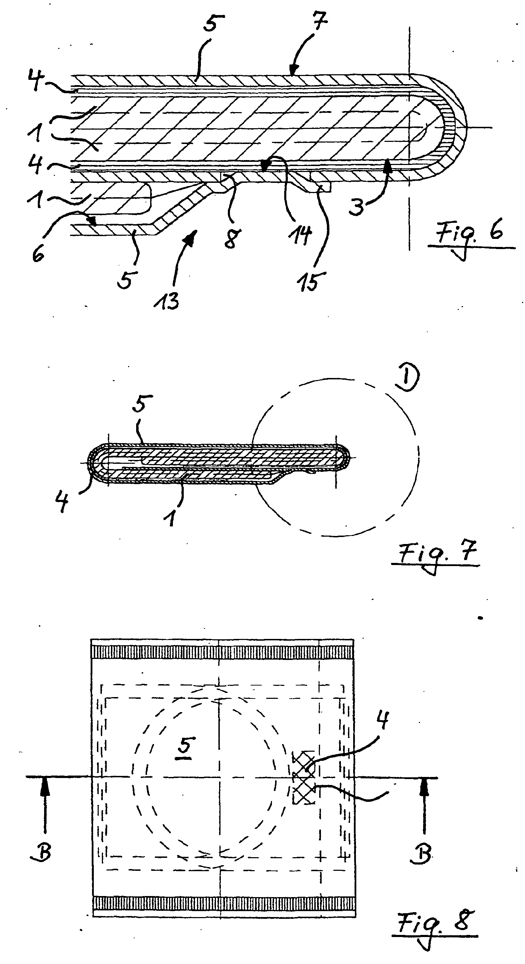

Die Fig. 6, 7 und 8 zeigen die fertig verpackte Slipeinlage 1, die in Fig. 8 in gestrichelten Linien dargestellt ist. Fig. 6 zeigt das vergrößerte Detail D gemäß Fig. 7. Wie deutlich im unteren Teil der Fig. 6 zu sehen ist, ragt das eine Ende 13 der Verpackungsfolie 5 über das zugehörige Ende der Slipeinlage 1 hinaus. Dadurch ist es möglich, dass ein Flächenbereich 14 der Innenseite 6 des hinausragenden Endes 13 der Verpackungsfolie 5 durch den Durchbruch 8 hindurch mit der Wäscheseite 3 der Slipeinlage 1 verklebt wird. Wie insbesondere in Fig. 6 zu erkennen, erfolgt dieses Verkleben mit Hilfe desjenigen Teils der Klebemittelschicht 4, die durch den Durchbruch 8 hindurch zugänglich ist. Da die Klebemittelschicht 4 die Verpackungsfolie 5 lösbar mit der Slipeinlage 1 verbindet, ist es auch ohne Weiteres möglich, den Flächenbereich 14 des Endes 13 der Verpackungsfolie 5 zum Entnehmen der Slipeinlage 1 aus der Verpackungsfolie 5 von der Wäscheseite 3 zu lösen. Hierzu braucht lediglich der vorzugsweise auf der Außenseite 7 der Verpackungsfolie 5 aufliegende Endbereich 15 des Endes 13 der Verpakkungsfolie 5 von Hand ergriffen und gezogen zu werden. Die Kante des Endbereichs 15 ist auch in Fig. 8 zu erkennen, wo sie als von oben nach unten verlaufende, gestrichelte Linie dargestellt ist.

Die beschriebene Ausführungsform zeigt deutlich, dass die vorliegende Erfindung in vorteilhafter Weise von der ohnehin auf die Wäscheseite 3 aufzutragenden Klebemittelschicht 4 Gebrauch macht. Der erfindunsgemäß vorgesehene Durchbruch 8 ermöglicht in einfacher Weise die Befestigung des Flächenbereichs 14 der Innenseite 6 der Verpackungsfolie 5 an der Wäscheseite 3 der Slipeinlage 1 und somit das sichere Verpacken der Slipeinlage 1. Darüber hinaus wird die Slipeinlage 1 hygienisch versiegelt von der Verpackungsfolie 5 umgeben.

## Patentansprüche

1. Verpacktes Hygieneprodukt zur Aufnahme von Körperflüssigkeiten und/oder -ausscheidungen umfassend
- das Hygieneprodukt (1), welches eine dem Körper zugewandte Körperseite (2) sowie eine von dem Körper abgewandte Wäscheseite (3) aufweist und zum Verpacken zusammenleg- oder -rollbar ist, wobei sich an der Wäscheseite (3) wenigstens teilweise ein Klebemittel (4) befindet, und
- ein Verpackungsmaterial (5), welches eine dem Hygieneprodukt (1) zugewandte Innenseite (6) und eine von dem Hygieneprodukt (1) abgewandte Außenseite (7) aufweist, wobei die Wäscheseite (3) des Hygieneprodukts (1) mit Hilfe des Klebemittels (4) an der Innenseite (6) befestigt ist,
**dadurch gekennzeichnet, dass**
das Verpackungsmaterial (5) wenigstens einen Durchbruch (8) aufweist, durch den hindurch ein Flächenbereich (14) der Innenseite (6) mit Hilfe eines Teils des Klebemittels (4) an der Wäscheseite (3) befestigbar ist, um das Hygieneprodukt (1) in verpacktem Zustand zu halten.

2. Verpacktes Hygieneprodukt nach Anspruch 1,
**dadurch gekennzeichnet, dass**
der Durchbruch (8) eine längliche Form aufweist.

3. Verpacktes Hygieneprodukt nach Anspruch 2,
**dadurch gekennzeichnet, dass**
das Hygieneprodukt (1) eine längliche Form aufweist und sich die Längsachse (9) des Durchbruchs (8) im Wesentlichen quer zu der Längsachse (10) des Hygieneprodukts (1) erstreckt.

4. Verpacktes Hygieneprodukt nach Anspruch 2,
**dadurch gekennzeichnet, dass**
das Hygieneprodukt (1) eine längliche Form aufweist und sich die Längsachse (9) des Durchbruchs (8) im Wesentlichen parallel zu der Längsachse (10) des Hygieneprodukts (1) erstreckt.

5. Verpacktes Hygieneprodukt nach einem der vorangehenden Ansprüche,
**dadurch gekennzeichnet, dass**
sich der Durchbruch (8) über die gesamte Breite (B) des Hygieneprodukts (1) oder des zusammengelegten Hygieneprodukts (1) erstreckt.

6. Verpacktes Hygieneprodukt nach einem der vorangehenden Ansprüche,
**dadurch gekennzeichnet, dass**
mehrere Durchbrüche (8) vorgesehen sind.

7. Verpacktes Hygieneprodukt nach Anspruch 6,
**dadurch gekennzeichnet, dass**
die Durchbrüche (8) in der Art einer Perforierung in einer geraden Linie oder in einer gekrümmten Kurve angeordnet sind.

8. Verpacktes Hygieneprodukt nach einem der vorangehenden Ansprüche,
**dadurch gekennzeichnet, dass**
das Klebemittel (4) mittels Kontaktauftrag aufbringbarer Adhäsivleim ist.

9. Verpacktes Hygieneprodukt nach einem der Ansprüche 1 bis 7,
**dadurch gekennzeichnet, dass**
das Klebemittel (4) mittels Sprühauftrag aufbringbarer Adhäsivleimleim ist.

10. Verfahren zur Herstellung eines verpackten Hygieneprodukts (1) zur Aufnahme von Körperflüssigkeiten und/oder -ausscheidungen umfassend die folgenden Schritte:
- Bereitstellen des Hygieneprodukts (1) mit einer dem Körper zugewandten Körperseite (2) sowie einer von dem Körper abgewandten Wäscheseite (3), und
- Bereitstellen eines Verpackungsmaterials (5) mit einer Innenseite (6) und einer Außenseite (7),
- wobei die Wäscheseite (3) des Hygieneprodukts (1) mit Hilfe eines Klebemittels (4) an der Innenseite (6) des Verpackungsmaterials (5) befestigt und das Hygieneprodukt (1) zusammen mit dem Verpackungsmaterial (5) zusammengelegt oder -gerollt wird,
**dadurch gekennzeichnet, dass**
in das Verpackungsmaterial (5) wenigstens ein Durchbruch (8) eingebracht wird und durch den Durchbruch (8) hindurch ein Flächenbereich (14) der Innenseite (6) mit Hilfe eines Teils des Klebemittels (4) an der Wäscheseite (3) befestigt wird, um das Hygieneprodukt (1) in verpacktem Zustand zu halten.

11. Verfahren nach Anspruch 10,
**dadurch gekennzeichnet, dass**
das Einbringen des wenigstens einen Durchbruchs (8) vor dem Befestigen der Wäscheseite (3) an der Innenseite (6) erfolgt.

## Claims

1. Packaged personal hygiene product to absorb bodily fluids and/or excretions, comprising
- the personal hygiene product (1) exhibiting a body side (2) facing toward the body and an undergarment side (3) facing away from the body and which can be folded or rolled for packaging, wherein there is an adhesive (4) at least on a part of the undergarment side (3), and
- packaging material (5) exhibiting an inner surface (6) facing toward the personal hygiene product (1) and an outer surface (7) facing away from the personal hygiene product (1) wherein the undergarment side (3) of the personal hygiene product (1) is affixed to the inner surface (6) by means of the adhesive (4),
**characterized by**
the packaging material (5) exhibiting at least one through opening (8) through which a flat surface area (14) of the inner surface (6) can be affixed to the undergarment side (3) with the help of a part of the adhesive (4) in order to keep the personal hygiene product (1) in its packed state.

2. Packaged personal hygiene product in accordance with Claim 1,
**characterized by**
the through opening (8) exhibiting an elongated shape.

3. Packaged personal hygiene product in accordance with Claim 2,
**characterized by**
the personal hygiene product (1) exhibiting an elongated shape and the longitudinal axis (9) of the through opening (8) running essentially perpendicular to the longitudinal axis (10) of the personal hygiene product (1).

4. Packaged personal hygiene product in accordance with Claim 2,
**characterized by**
the personal hygiene product (1) exhibiting an elongated shape and the longitudinal axis (9) of the through opening (8) running essentially parallel to the longitudinal axis (10) of the personal hygiene product (1).

5. Packaged personal hygiene product in accordance with one of the foregoing claims,
**characterized by**
the through opening (8) extending across the entire width (B) of the personal hygiene product (1) or of the folded personal hygiene product (1).

6. Packaged personal hygiene product in accordance with one of the foregoing claims,
**characterized by**
a plurality of through openings (8) being provided.

7. Packaged personal hygiene product in accordance with Claim 6,
**characterized by**
the through openings (8) being arranged so as to create a perforation in a straight line or in a curved line.

8. Packaged personal hygiene product in accordance with one of the foregoing claims,
**characterized by**
the adhesive (4) being an adhesive glue that can be applied by way of contact application.

9. Packaged personal hygiene product in accordance with one of the Claims 1 to 7,
**characterized by**
the adhesive (4) being an adhesive glue that can be applied by means of spray application.

10. Process to manufacture a packaged personal hygiene product (1) to absorb bodily fluids and/or excretions, comprising the following steps:
- readying for processing the personal hygiene product (1) exhibiting a body side (2) facing toward the body and an undergarment side (3) facing away from the body, and
- readying for processing packaging material (5) exhibiting an inner surface (6) and an outer surface (7),
- wherein the undergarment side (3) of the personal hygiene product (1) is affixed with the help of an adhesive (4) to the inner surface (6) of the packaging material (5) and the personal hygiene product (1) is folded or rolled together with the packaging material (5),
**characterized by**
at least one through opening (8) being incorporated into the packaging material (5) and by a flat surface area (14) of the inner surface (6) being affixed to the undergarment side (3) through the through opening (8) with the help of a part of the adhesive (4) in order to keep the personal hygiene product (1) in its packed state.

11. Process in accordance with Claim 10,
**characterized by**
the incorporation of the minimum of one through opening (8) being effected prior to the affixing of the undergarment side (3) to the inner surface (6).

## Revendications

1. Produit hygiénique emballé destiné à l'absorption de liquides biologiques et / ou d'éliminations corporelles, comprenant
- le produit hygiénique (1), qui présente une face « corps » (2) orientée vers le corps et une face « linge » (3) opposée au corps et qui peut être plié ou enroulé pour être emballé, une substance collante (4) se trouvant, du moins partiellement, sur la face « linge » (3), et
- un matériel d'emballage (5), qui présente une face intérieure (6) orientée vers le produit hygiénique (1) et une face extérieure (7) opposée au produit hygiénique (1), la face « linge » (3) du produit hygiénique (1) étant fixée à la face intérieure (6) à l'aide de la substance collante (4),
**caractérisé par le fait que**
le matériel d'emballage (5) présente au moins une ouverture (8) à travers laquelle une surface partielle (14) de la face intérieure (6) peut être fixée à la face « linge » (3) à l'aide d'une partie de la substance collante (4) afin de maintenir le produit hygiénique (1) à l'état emballé.

2. Produit hygiénique emballé d'après la revendication 1,
**caractérisé par le fait que**
l'ouverture (8) présente une forme longitudinale.

3. Produit hygiénique emballé d'après la revendication 2,
**caractérisé par le fait que**
le produit hygiénique (1) présente une forme longitudinale et que l'axe longitudinal (9) de l'ouverture (8) s'étend essentiellement dans le sens transversal par rapport à l'axe longitudinal (10) du produit hygiénique (1).

4. Produit hygiénique emballé d'après la revendication 2,
**caractérisé par le fait que**
le produit hygiénique (1) présente une forme longitudinale et que l'axe longitudinal (9) de l'ouverture (8) s'étend essentiellement parallèlement à l'axe longitudinal (10) du produit hygiénique (1).

5. Produit hygiénique emballé d'après l'une des revendications précédentes,
**caractérisé par le fait que**
l'ouverture (8) s'étend sur toute la largeur (B) du produit hygiénique (1) ou du produit hygiénique (1) replié.

6. Produit hygiénique emballé d'après l'une des revendications précédentes,
**caractérisé par le fait que**
plusieurs ouvertures (8) sont prévues.

7. Produit hygiénique emballé d'après la revendication 6,
**caractérisé par le fait que**
les ouvertures (8) sont disposées dans le style d'une perforation sur une ligne droite ou sur une courbe.

8. Produit hygiénique emballé d'après l'une des revendications précédentes,
**caractérisé par le fait que**
la substance collante (4) est une colle adhésive applicable par contact.

9. Produit hygiénique emballé d'après l'une des revendications 1 à 7,
**caractérisé par le fait que**
la substance collante (4) est une colle adhésive applicable par pulvérisation.

10. Procédé de fabrication d'un produit hygiénique (1) emballé destiné à l'absorption de liquides biologiques et / ou d'éliminations corporelles, comprenant les étapes suivantes :
- mise à disposition du produit hygiénique (1) avec une face « corps » (2) orientée vers le corps ainsi qu'une face « linge » (3) opposée au corps,
et
- mise à disposition du matériel d'emballage (5) avec une face intérieure (6) et une face extérieure (7),
- la face « linge » (3) du produit hygiénique (1) étant fixée à la face intérieure (6) du matériel d'emballage (5) à l'aide d'une substance collante (4) et le produit hygiénique (1) étant plié ou enroulé avec le matériel d'emballage (5),
**caractérisé par le fait que**
au moins une ouverture (8) est appliquée dans le matériel d'emballage (5) et qu'à travers cette ouverture (8), une surface partielle (14) de la face intérieure (6) peut être fixée à la face « linge » (3) à l'aide d'une partie de la substance collante (4) afin de maintenir le produit hygiénique (1) à l'état emballé.

11. Procédé d'après la revendication 10,
**caractérisé par le fait que**
l'application d'au moins une ouverture (8) a lieu avant la fixation de la face « linge » (3) sur la face intérieure (6).
